# EUROPEAN PATENT APPLICATION

(11) **EP 4 748 824 A1**
(43) Date of publication of application: **27.05.2026**
(21) Application number: 24306975.4
(22) Date of filing: 26.11.2024
(51) Int. Cl.: C07C 67/00, C07C 69/16, C07C 67/24

(54) **PROCESS OF PREPARATION OF METHYLENE DICARBOXYLATES**

(71) Applicant: Crackless Monomers Co., Ltd, Chiayi Hsien 621 (TW)
(72) Inventor: TCHAPLINSKI, Vladimir, 08193 Bellaterra, Barcelona (ES); BABA-AHMED, Abdelatif, 69492 Pierre-Bénite Cedex (FR)
(74) Representative: Arkema Patent

(57) **Abstract**

The present invention relates to a process of preparation of a compound having formula (I):

R¹-C(=O)-O-CH₂-O-C(=O)-R² (I)

wherein R¹ and R² represent, independently of each other, an alkyl, a cycloalkyl or an aryl group, said alkyl, cycloalkyl or aryl group being optionally substituted;
said process comprising a step i) of reacting:
∘ a compound A selected from the group consisting of: trioxane, polyoxymethylene deprived of hydroxyl end groups;
∘ a compound of formula (II):

R¹-C(=O)-O-C(=O)-R² (II)

and
∘ a perfluorosulfonic acid;

wherein step i) is carried out at a temperature T1 of at least 50°C,
wherein the molar ratio of compound of formula (II) : compound A ranges from 1 : 1 to 1.2 : 1.

## Description

### TECHNICAL FIELD

The present invention relates a process of preparation of methylene dicarboxylates, and the uses thereof.

### TECHNICAL BACKGROUND

Methylene dicarboxylates is a class of compounds which are diesters of methanediol. These compounds gained interest in the past as means of fungus and bacteria control in crops and animal feedstocks, and as acylation agents for plasticization and stabilization of polyoxymethylene. These compounds are also used as starting material for preparation of cyanoacrylates.

Some processes are known and consist of reacting solid paraformaldehyde with carboxylic acid anhydride and catalysts. However, there exist some drawbacks such as obtaining of high viscosity (which imply stirring issues) and exothermicity (which imply difficulty to control the reaction temperature). Besides, high catalyst loads are typically needed to complete the reaction. Finally, the resulting products need to be further purified by neutralization steps, and further complex fractional distillation to get pure products. At industrial scale, these processes are thus complex, time consuming, energy consuming, generating of wastes due to formation of by-products and use of basic neutralizers at the work up step.

There is thus a need for a new process that would overcome at least partially one or several of the above-mentioned drawbacks.

More particularly, there is a need for a process of preparation of methylene dicarboxylates exhibiting high conversion of all starting materials, high yield and high purity.

There is a need for a process of preparation of methylene dicarboxylates which can be implemented continuously, with low load of catalyst, controlled exothermicity, and being fast.

### DESCRIPTION OF THE INVENTION

The present invention relates to a process of preparation of a compound having formula (I):

R¹-C(=O)-O-CH₂-O-C(=O)-R² (I)

wherein R¹ and R² represent, independently of each other, an alkyl, a cycloalkyl or an aryl group, said alkyl, cycloalkyl or aryl group being optionally substituted;
said process comprising a step i) of reacting:
   ∘ a compound A selected from the group consisting of: trioxane, polyoxymethylene deprived of hydroxyl end groups;
   ∘ a compound of formula (II):

      R¹-C(=O)-O-C(=O)-R² (II)

      and
   ∘ a perfluorosulfonic acid;
wherein step i) is carried out at a temperature T1 of at least 50°C,
wherein the molar ratio of compound of formula (II) : compound A ranges from 1 : 1 to 1.2 : 1.

Preferably, the molar ratio of compound of formula (II) : compound A ranges from 1 : 1 to 1.1 : 1, and even more preferably from 1:1 to 1.05 : 1.

### Compound of formula (I)

The compounds of formula (I) are preferably those wherein:
- R¹ represents an alkyl optionally substituted; and
- R² represents an alkyl optionally substituted.

Preferred compounds of formula (I) are those wherein:
- R¹ represents an alkyl comprising from 1 to 10 carbon atoms; and
- R² represents an alkyl comprising from 1 to 10 carbon atoms.

Representative alkyl groups for R¹ and R², may include, independently of each other, methyl, ethyl, n-propyl, isopropyl, n-butyl, isobutyl, n-pentyl, 2-methylbutyl, hexyl, and isohexyl.

Preferably, in the compounds of formula (I), R¹ and R² are identical.

More preferably, the compound of formula (I) is:

CH₃-C(=O)-O-CH₂-O-C(=O)-CH₃

### Compound of formula (II)

The compounds of formula (II) are preferably those wherein:
- R¹ represents an alkyl optionally substituted; and
- R² represents an alkyl optionally substituted.

Preferred compounds of formula (II) are those wherein:
- R¹ represents an alkyl comprising from 1 to 10 carbon atoms; and
- R² represents an alkyl comprising from 1 to 10 carbon atoms.

Representative alkyl groups for R¹ and R², may include, independently of each other, methyl, ethyl, n-propyl, isopropyl, n-butyl, isobutyl, n-pentyl, 2-methylbutyl, hexyl, and isohexyl.

Preferably, in the compounds of formula (II), R¹ and R² are identical.

More preferably, the compound of formula (II) is:

CH₃-C(=O)-O-C(=O)-CH₃

Compound of formula (II) is typically carboxylic acid anhydride, and may be prepared from carboxylic acid by methods known in the art. Compound of formula (II) may also be commercially available from Sigma Aldrich.

### Compound A

The compound A is selected from the group consisting of: trioxane, polyoxymethylene deprived of hydroxyl end groups.

Preferably, the compound A is trioxane, and more preferably 1,3,5-trioxane.

Trioxane is for example commercially available from Sigma Aldrich.

Polyoxymethylene deprived of hydroxyl end groups is for example POM (Delrin) commercialized by Dupont.

### Perfluorosulfonic acid

Perfluorosulfonic acid has typically the following formula: C*ₙ*F_{2*n+*1}SO₃H wherein n is an integer.

Preferably, the perfluorosulfonic acid is selected from the group consisting of: trifluoromethanesulfonic acid, perfluoroethanesulfonic acid, perfluoropropanesulfonic acid, perfluorobutanesulfonic acid, perfluoropentanesulfonic acid, perfluorohexanesulfonic acid, perfluoroheptanesulfonic acid, perfluorooctanesulfonic acid, perfluorononanesulfonic acid, perfluorodecanesulfonic acid.

More preferably, the perfluorosulfonic acid is trifluoromethanesulfonic acid.

The perfluorosulfonic acid is preferably used in a mol% ranging from 0.01 mol% to 0.1, more preferably from 0.01 to 0.05 mol% relative to 100mol% of the compound of formula (II).

The perfluorosulfonic acid is typically a catalyst that will not react with other compounds, and will be recovered at the end of the reaction.

### Steps

Step i) may be carried out solventless or in presence of solvent. If present, the solvent may be selected from polar solvents such as for example xylene, dibutyl ether, ethylene glycol diacetate, or mixtures thereof.

Preferably, step i) is conducted solventless.

Step i) may be carried out under inert atmosphere, for example in presence of nitrogen, argon, or helium.

Preferably, step i) is carried out in a reactor.

Step i) may last from 1 hour to 24 hours, preferably from 1 hour to 10 hours, and even more preferably from 1 hour to 4 hours.

Step i) is preferably carried out at a temperature T1 greater than or equal to 80°C, even more preferably at a temperature T1 ranging from 100°C to 200°C.

Step i) may be carried out in presence of a compound of formula (IV):

R'-C(=O)-OH (IV)

wherein R¹ is an alkyl, a cycloalkyl or an aryl group, said alkyl, cycloalkyl or aryl group being optionally substituted.

The compounds of formula (IV) are preferably those wherein R¹ represents an alkyl optionally substituted, more preferably wherein R¹ represents an alkyl comprising from 1 to 10 carbon atoms.

Preferably, R¹ in the compound of formula (IV) is identical to R¹ in the compounds of formula (I) and (II).

More preferably, the compound of formula (IV) is:

CH₃-C(=O)-OH

Preferably the content of the compound of formula (IV) ranges from 2 wt.% to 10 wt.% based on the total mass of the reaction medium. More preferably, it ranges from 4 wt% to 6 wt% based on the total mass of the reaction medium.

Step i) is preferably carried out without cyanoacetate compound.

Step i) is preferably carried out without carbon monoxide.

Step i) is preferably carried out without Group VIII noble transition metal compound.

Step i) is preferably carried out without the presence of additional compounds other than: compound A, compound of formula (II), perfluorosulfonic acid, and optionally compound of formula (IV).

The process may be carried out in a single reactor, or in a cascade of reactors. The reactor may be a tubular reactor.

The process may be carried out continuously or by batch.

The process of the invention preferably comprises, (prior to or simultaneously to step i)), a step i') of contacting the compound of formula (II), the compound A, and the perfluorosulfonic acid.

This step i') may comprise:
- contacting the perfluorosulfonic acid with a mixture comprising the compound of formula (II) and the compound A (the contacting step may be for example the addition of the perfluorosulfonic acid into the mixture, or the addition of the mixture into the perfluorosulfonic acid);
   or
- contacting the compound A with a mixture comprising the compound of formula (II) and the perfluorosulfonic acid (the contacting step may be for example the addition of the compound A into the mixture, or the addition of the mixture into the compound A);
   or
- contacting simultaneously the compound A, the compound of formula (II) and the perfluorosulfonic acid (for example in case each compound is fed separately to a reactor).

The contacting step i') may be carried out at a temperature T2 lower than or equal to T1. If the temperature T2 is different from T1, then it may range from 20°C to 49°C, preferably from 20°C to 30°C.

If the temperature T2 is different from T1, then the process preferably comprises a heating step ii') from T2 to T1.

Step i) preferably leads to a composition comprising the compound of formula (I).

Advantageously, the process allows the conversion of at least 90% of the compound A to a compound of formula (I). Even more preferably, the process allows conversion of at least 96% of the compound A to a compound of formula (I).

Advantageously, the process allows the conversion of at least 80% of the compound A to a compound of formula (I) after 1 hour.

The process may comprise a subsequent step ii), after step i), of recovery of the compound of formula (I).

Step ii) preferably comprises contacting the composition obtained after step i) with an adsorbent material.

The adsorbent material may be a solid material of basic, acidic or neutral character insoluble in the reaction mixture.

Preferably, the adsorbent material is a clay, a zeolite, alumina, silica gel, activated carbon, magnesium silicate (e. g. magnesol), calcium carbonate, calcium stearate, basic ion-exchange resin.

More preferably, the adsorbent material is a clay (preferably a bentonite) or inorganic silicate.

The step ii) is preferably carried out at a temperature ranging from 20°C to 30°C, even more preferably at 23°C.

The step ii) advantageously allows to separate the perfluorosulfonic acid from the compound of formula (I).

The compound of formula (I) obtained after the recovery step ii) has advantageously a high purity, preferably a purity of at least 90%, more preferably of at least 92%.

The compound of formula (I) preferably comprises less than 5 wt %, more preferably less than 2 wt % of impurities selected from compound of formula (II), compound of formula (III):

R¹-C(=O)-O-CH₂-O-CH₂-O-C(=O)-R² (III)

wherein R¹ and R² are as defined above,
and mixtures thereof.

The process may comprise an intermediate step between step i) and step ii), comprising submitting the medium obtained after step i) to moisture. It may be done by mixing at a temperature ranging from 20°C to 80°C.

Advantageously, the process does not comprise a purification step after step ii). More preferably, the process does not comprise a distillation step after step ii).

The process also preferably does not comprise neutralization step after step i) and/or step ii).

The process according to the invention exhibits at least one of the following advantages:
- atom economic way to lead to a compound of formula (I) with reduced level of impurities;
- high conversion of all the starting material;
- high yield;
- high purity of the compound of formula (I) that does not require additional distillation step, thus it decreases the carbon footprint compared to other processes involving fractional distillation;
- little consumption of catalyst;
- energy saving process;
- safe process with no issue of exothermicity;
- fast process.

The present invention also relates to the compound of formula (I) obtained by the process as disclosed herein.

The present invention relates to a composition comprising the compound of formula (I) obtained by the process as disclosed herein.

The present invention also relates to the use of a compound of formula (I) obtained by the process as disclosed herein, for preparing cyanoacrylate.

The ranges disclosed in this description include both the lower and the upper limit thereof. For example, the expressions "ranging from x to y" or "between x and y" comprises the limits x and y.

### Experimental part

### Example 1 : preparation of methylene diacetate (sulfuric acid, comparative)

30 g of trioxane (from Sigma Aldrich) were dissolved in 102 g of AczO (acetic anhydride from Sigma Aldrich) in a 250 mL flask not immersed in any bath in order to evaluate exothermicity of the process. During the dissolving process, the temperature of the solution decreased from 23°C to 8°C. The cold mixture was immersed in oil bath, warmed up to 23°C and kept at 23°C during 1h. Analysis of this solution showed no reaction.

After that, the oil bath was removed and 0.49 g of sulfuric acid (0.5 mol%, from Scharlau) dissolved in 6.8 g of acetic acid (corresponding to 5% of final mass, from Thermo Fisher) were added in small drops into the stirred solution. At addition of the catalyst, the exothermic reaction has started and the temperature of the mixture rose to 82 °C.

After the exothermicity of the reaction was over, the mixture was immersed into oil bath again and heated at 140°C for 4 h. The mixture was analysed by means of NMR after 0, 1, 2 and 4h of heating.

Example 2 was then repeated but with 1 mol% of sulfuric acid (comparative).

Example 3 was then repeated but with 0.02 mol% (~200ppm) of triflic acid (invention).

Figure 1 discloses the methylene diacetate content (wt.%) versus the time in hours (x- axis)

According to Figure 1, example 1 (comparative) catalysed by 0.5 mol % of sulfuric acid (SA) leads to around 50 wt.% of methylene diacetate after one hour, and less than 80 wt.% after 4 hours. On the contrary, example 3 (200 ppm triflic acid: TFA) advantageously leads to a high weight content percentage (wt.%) of methylene diacetate after only one hour (more than 80 wt.%) and with much lower quantity of catalyst (200ppm by weight). The reaction is over between 1h and 2h with example 3. Thus, the reaction of example catalysed by triflic acid is advantageously faster with low amount of catalyst.

Figure 2 discloses weight content (wt.%) of dioxymethylene diacetate (DOD) with time (in hours)

Figure 2 shows the consumption of residual DOD in the Example 2 (with 1 mol% of SA - comparative) and Example 3 (with ~200 ppm TFA - invention).

In the reaction catalysed by 1% mol of SA (example 2 - comparative) the residual DOD level falls to 2.5 wt.% after 4 h of heating and in the reaction catalysed by 200ppm of TFA (example 3 - invention) it already falls under this value between only 1h and 2h.

### Example 4 : work-up of Example 3 by adsorbing filtration

The reaction mixture containing prepared methylene diacetate, obtained by catalysis with 200 ppm TFA in Example 3 was filtered through adsorbing material without any additional work up:
The mixture of Example 3 was slowly filtered through a preformed plug of 2.8 g (2 wt. % ref. to the total mass of reaction mixture) of basic Bentonite clay (from Sigma Aldrich) using a small Chemrus disposable filter funnel as support (Polypropylene body, pore size 10 microns). Under 200mbar vacuum the filtration took 6h in total. The filtrate was pale brown but much clearer than the crude mixture which contained some black particles.

NMR analysis of the filtrate corresponds to the NMR spectra of the reaction mixture before filtration.

### Example 5 : work-up of Example 3 by adsorbing filtration

The reaction mixture containing prepared methylene diacetate, obtained by catalysis with 200 ppm TFA in Example 3 was filtered through adsorbing material without any additional work up:
The mixture of Example 3 was slowly filtered through a preformed plug of 2.8 g (2 wt. % ref. to the total mass of reaction mixture) of Magnesol (from Dallas Group of America) using a small Chemrus disposable filter funnel as support (Polypropylene body, pore size 10 microns). Under 200mbar vacuum the filtration took 30min in total. The filtrate was pale brown but much clearer than the crude mixture which contained some black particles.

NMR analysis of the filtrate corresponds to the NMR spectra of the reaction mixture before filtration.

### Example 6 : preparation of 2-methoxyethyl cyanoacrylate (MECA)

The synthesis of 2-methoxyethyl cyanoacrylate (MECA) is carried out from 2-methoxyethyl cyanoacetate using the corresponding methylene diacetate according to the method disclosed in EP2927209 (in particular in example 1), at a 10 mmol scale in presence of radical stabilizers known to the skilled in the art.

The ingredients of the reaction are as follows:

| | | |
|---|---|---|
| 2-methoxyethyl cyanoacetate | 1.43 g | (10 mmol) |
| Methylene diacetate | 2.64 g | (20 mmol) |
| Methanesulfonic acid | 144 mg | (1.5 mmol) |
| Piperazine | 21.5 mg | (0.25 mmol) |

The reactions with 3 different methylene diacetate samples (6a, 6b, 6c) were performed at 125 °C and the crude reaction mixtures were analyzed by means of NMR after 90 min of reaction.
In example 6a: the methylene diacetate supplied by CMC, which was purified by fractional distillation (comparative)
In example 6b: the methylene diacetate obtained in example 4 (after adsorbing filtration, invention)
In example 6c: the methylene diacetate obtained in example 5 (after adsorbing filtration, invention)

| **Example** | | |
|---|---|---|
| | Selectivity : the content of MECA produced with respect to the initial 2-methoxyethyl cyanoacetate | Unreacted 2-methoxyethyl cyanoacetate |
| **6a (comparative)** | 83% | 3.5% |
| **6b (invention)** | 86% | 1.6% |
| **6c (invention)** | 86% | 2.1% |

It can be seen from this table that the conversion of 2-methoxyethyl cyanoacetate is advantageously higher in examples 6b and 6c (invention) compared to example 6a (comparative): indeed the content of unreacted 2-methoxyethyl cyanoacetate being lower at 1.6% (6b), 2.1% (6c) compared to 3.5% (6a).

Besides, the degree of selectivity is advantageously higher in examples 6b and 6c (invention) (86%) compared to example 6a (83%) : the reactivity of methylene diacetate samples purified by only contacting step with adsorbent material is better than that of methylene diacetate obtained and purified by existing method (fractional distillation).

## Claims

1. Process of preparation of a compound having formula (I):
R¹-C(=O)-O-CH₂-O-C(=O)-R² (I)
wherein R¹ and R² represent, independently of each other, an alkyl, a cycloalkyl or an aryl group, said alkyl, cycloalkyl or aryl group being optionally substituted;
said process comprising a step i) of reacting:
∘ a compound A selected from the group consisting of: trioxane, polyoxymethylene deprived of hydroxyl end groups;
∘ a compound of formula (II):
R¹-C(=O)-O-C(=O)-R² (II)
and
∘ a perfluorosulfonic acid;
wherein step i) is carried out at a temperature T1 of at least 50°C,
wherein the molar ratio of compound of formula (II) : compound A ranges from 1 : 1 to 1.2 : 1.

2. Process according to claim 1, wherein the molar ratio of compound of formula (II) : compound A ranges from 1 : 1 to 1.1 : 1.

3. Process according to anyone of claim 1 or claim 2, wherein the molar ratio of compound of formula (II) : compound A ranges from 1:1 to 1.05 : 1.

4. Process according to anyone of claims 1 to 3, wherein step i) is carried out at a temperature T1 greater than or equal to 80°C, preferably at a temperature T1 ranging from 100°C to 200°C.

5. Process according to anyone of claims 1 to 4, wherein step i) is carried out in presence of a compound of formula (IV):
R'-C(=O)-OH (IV)
wherein R¹ is an alkyl, a cycloalkyl or an aryl group, said alkyl, cycloalkyl or aryl group being optionally substituted.

6. Process according to anyone of claims 1 to 5, wherein the content of the compound of formula (IV) ranges from 2 wt.% to 10 wt.% based on the total mass of the reaction medium, preferably, it ranges from 4 wt% to 6 wt% based on the total mass of the reaction medium.

7. Process according to anyone of claims 1 to 6, wherein the compound of formula (I) is selected from those wherein:
- R¹ represents an alkyl comprising from 1 to 10 carbon atoms; and
- R² represents an alkyl comprising from 1 to 10 carbon atoms.

8. Process according to anyone of claims 1 to 7, wherein the compound of formula (I) is:
CH₃-C(=O)-O-CH₂-O-C(=O)-CH₃

9. Process according to anyone of claims 1 to 8, wherein the compound A is trioxane.

10. Process according to anyone of claims 1 to 9, wherein the perfluorosulfonic acid is selected from the group consisting of: trifluoromethanesulfonic acid, perfluoropropanesulfonic acid, perfluoroethanesulfonic acid, perfluorobutanesulfonic acid, perfluoropentanesulfonic acid, perfluorohexanesulfonic acid, perfluoroheptanesulfonic acid, perfluorooctanesulfonic acid, perfluorononanesulfonic acid, perfluorodecanesulfonic acid.

11. Process according to anyone of claims 1 to 10, wherein the perfluorosulfonic acid is trifluoromethanesulfonic acid.

12. Process according to anyone of claims 1 to 11, wherein the perfluorosulfonic acid is used in a mol% ranging from 0.01 mol% to 0.05 mol% relative to the compound of formula (II).

13. Process according to anyone of claims 1 to 12, wherein the process allows the conversion of at least 90% of the compound A to a compound of formula (I).

14. Process according to anyone of claims 1 to 13, wherein it further comprises a subsequent step ii), after step i), of recovery of the compound of formula (I).

15. Process according to anyone of claims 1 to 14, wherein step ii) comprises contacting the composition obtained after step i) with an adsorbent material, preferably selected from a clay, a zeolite, alumina, silica gel, activated carbon, magnesium silicate, calcium carbonate, calcium stearate, basic ion-exchange resin, more preferably, the adsorbent material being a clay or inorganic silicate.

16. Process according to anyone of claims 1 to 15, wherein the compound of formula (I) obtained after the recovery step has a purity of at least 90%, preferably of at least 92%.

17. Process according to anyone of claims 1 to 16, wherein said process does not comprise a purification step after step ii).

18. Compound of formula (I) obtained by the process according to anyone of claims 1 to 17.

19. Use of the compound of formula (I) according to claim 18, for preparing cyanoacrylate.
